Europäisches Patentamt

European Patent Office

Office européen des brevets

(11) Veröffentlichungsnummer: **0 430 872 B1**

(12) **EUROPÄISCHE PATENTSCHRIFT**

(45) Veröffentlichungstag der Patentschrift: 05.04.95

(51) Int. Cl.⁶: **C23C 14/32**, C23C 14/06, C23C 14/02, B29C 45/00, A61B 17/00, A61F 2/00

(21) Anmeldenummer: 90810861.6

(22) Anmeldetag: 09.11.90

(54) **Werkzeug oder Instrument mit einer verschleissresistenten Hartschicht zum Be- oder Verarbeiten von organischem Material.**

(30) Priorität: 22.11.89 CH 4189/89

(43) Veröffentlichungstag der Anmeldung:
05.06.91 Patentblatt 91/23

(45) Bekanntmachung des Hinweises auf die Patenterteilung:
05.04.95 Patentblatt 95/14

(84) Benannte Vertragsstaaten:
AT CH DE ES FR GB IT LI NL SE

(56) Entgegenhaltungen:
EP-A- 0 043 781     EP-A- 0 166 708
EP-A- 0 209 137     EP-A- 0 385 283
EP-A- 0 413 853     FR-A- 2 393 079

4EME COLLOOUE INTERNATIONAL SUR LES PLASMAS ET LA PULVERISATION CATHODI-OUE, C.I.P. 1982, Nice, 13. - 17. September 1982, VIDE, Couches Minces, Nr. 212, (Suppl.), Seiten 191-199; N.A.G. AHMED et al.: "The properties of ion-plated alumini-um/aluminium-oxide coatings deposited in a pulsed oxygen gas"

(73) Patentinhaber: **BALZERS AKTIENGESELL-SCHAFT**

**FL-9496 Balzers (LI)**

(72) Erfinder: **Schulz, Hans Dr.**
**Alte Churerstrasse 792**
**LI-9496 Balzers (LI)**
Erfinder: **Daxinger, Helmut**
**Melserstrasse**
**CH-7323 Wangs (CH)**
Erfinder: **Bergmann, Erich Dr.**
**Sarganserstrasse 58**
**CH-8887 Mels (CH)**
Erfinder: **Ramm Jürgen Dr.**
**Unterdorf 72b**
**CH-7306 Flaesch (CH)**

PATENT ABSTRACTS OF JAPAN, Band 13, Nr. 134 (C-581)[3482], 4. April 1989; & JP-A-63 297 552

PATENT ABSTRACTS OF JAPAN, Band 13, Nr. 134 (C-581)[3482], 4. April 1989; & JP-A-62 297 552

(74) Vertreter: **Keller, René, Dr. et al**
**Dr. R. Keller & Partner**
**Patentanwälte**
**Marktgasse 31**
**Postfach**
**CH-3000 Bern 7 (CH)**

## Beschreibung

Die Erfindung betrifft ein Werkzeug oder Instrument mit mindestens einer verschleißresistenten Hartschicht gemäß dem Oberbegriff des Patentanspruchs 1 und ein Verfahren zur Herstellung der Beschichtung gemäß dem Oberbegriff des Patentanspruchs 8.

Die bekannten Werkzeuge und Instrumente zum Bearbeiten von organischen Material benötigen aufgrund ihres Einsatzgebietes einen Grundkörper mit einer extrem hohe Zähigkeit. Stähle besitzen diese Eigenschaften; sie sind jedoch nicht korrosionsfest. Zum Schutz vor Korrosion wurden die bekannten Werkzeuge und Instrumente verchromt. Diese Chromschicht konnte den Grundkörper in agressiver Umgebung nur vorübergehend schützen; sie blätterte bereits nach kurzer Zeit ab.

Aus der DE-OS 36 39 469 ist ein anderes Schneid- und Formwerkzeug mit hoher Verschleißfestigkeit und Korrosionsminderung bekannt, welches mehrere Zwischenschichten und eine schwarze Deckschicht bestehend aus einer harten Kohlenstoffschicht (iC) mit eingelagerten Kristalliten des Karbids der Zwischenschicht hat. Die erste Schicht der Zwischenschicht besteht aus einem Element der Gruppe IVb oder Vb des Periodensystems (Titan, Zirkonium, Hafnium, Vanadium, Niob oder Tantal), die zweite Schicht aus einem Nitrid dieses Elements und die dritte Schicht aus einer Karbidschicht des gleichen Elements.

Die Korrosionsbeständigkeit der anderen bekannten Schneid- und Formwerkzeuge ist namentlich bei der Verarbeitung organischen Materials u. a. bei der Verwendung als Vulkanisier- und Kunststoffspritzgußform mangelhaft. Eine Schichtablösung beim Salzsprühnebeltest fand bereits nach 48 Stunden statt, d. h. derart beschichtete Werkzeuge und Instrumente sind nicht mehrfach spülmaschinenfest und auch nicht mehrfach sterilisierbar. Ebenfalls ist das Herstellungsverfahren, bei dem drei Zwischenschichten auf einen Grundkörper des Schneid- und Formwerkzeuges aufgebracht werden müssen, aufwendig. Die schwarze bis anthrazitfarbene Oberfläche ergibt kein ansprechendes Aussehen für Schneidwerkzeuge z. B. im Haushalt oder für chirurgische Instrumente.

In der EP-A 0 209 137 werden verschiedene Metalle, Legierungen, gesinterte Hochgeschwindigkeitsstähle, Keramiken, etc. durch aufwachsen lassen von kubischem Bornitrid auf einer Stickstoff enthaltenden Zwischenschicht (Trennschicht) beschichtet. Die beschichteten Gegenstände dienen als verschleißfeste Schneidwerkzeuge und Halbleitersubstrate. Damit das Bornitrid kubisch auf der Zwischenschicht aufwachsen kann, muß diese kristallin mit einer wohl definierten orientierten Kristalloberfläche ausgebildet sein.

Die bekannte Beschichtung ist sehr hart, da der beschichtete Gegenstand zur Bearbeitung von Materialien der Eisengruppe verwendet wird.

In der EP-A 0 043 781 wird eine mehrlagige Beschichtung mit einer Oberschichtenfolge und einer fakultativen Zwischenschicht beschrieben. Die Oberschichtenfolge weist abwechselnd eine stöchiometrischen und eine nicht-stöchiometrische Zusammensetzung mit ein- und demselben Übergangsmetall und ein- und demselben Nichtmetall auf. Die Zwischenschicht, sofern vorhanden, ist eine nichtmetallische Eisenverbindung, insbesondere $Fe_3N$.

Die Härte der äußersten Schicht beträgt mehr als 1000 Vickers und die Schichtdicke ist kleiner als 5 $\mu$m. Die Härte der Zwischenschicht mit einer Schichtdicke kleiner 5 $\mu$m ist größer als 500 Vikkers. Die unmittelbar auf dem Substrat aufliegende Schicht weist eine Härte größer 1000 Vickers und eine Schichtdicke kleiner 20 $\mu$m auf.

In der EP-A 0 166 708 wird auf einen Verbundgrundkörper bzw. gesinterten Grundkörper (compound body) mit einem CVD-Verfahren eine Diamantoberschicht und mit einem CVD- oder PVD-Verfahren eine keinen Diamant enthaltende Zwischenschicht aufgebracht. Die Schichtdicke der Oberschicht beträgt 0,1 bis 10 $\mu$m.

Das Material des Grundkörpers besteht aus Carbiden, Nitriden, Carbonitriden, Oxycarbiden, Boriden der Metalle der Gruppen IVb bis VIb, wie WC, TaC, Ti(C,0), (Ti, Mo) (C,N), $Mo_2C$, $Cr_3C2$, VC, MoC.

Die bekannte Zwischenschicht (Trennschicht) kann bestehen aus Carbiden, Nitriden, Carbonitriden, Oxycarbiden, Oxiden oder Boriden der Metalle der Gruppen IVb bis VIb, wie z. B. TiN, $Si_3N_4$, $Al_2O_3$, welche verallgemeinernd sich mit der Formel $Me(C_xN_yO_zB_t)$ beschreiben lassen, wobei Me ein Metall aus der Gruppe 4b bis 6b des Periodensystems und $0,45 < x + y + z + t < 2,1$ ist. Die Schichtdicke der Zwischenschicht beträgt 10 Å bis 10 $\mu$m.

Die bekannten beschichteten Gegenstände werden als Ziehsteine(-düsen), Dichtringe, Fadenführelemente, Fräswerkzeuge und Abdrehwerkzeuge für Holz, Plastiklaminat und Faserverbundwerkstoffe eingesetzt.

Der Erfindung liegt die Aufgabe zugrunde, eine haltbare, mehrfach spülmaschinenfeste bzw. mehrfach sterilisierbare und einfach herzustellende Beschichtung auf einem Werkzeug oder Instrument zu schaffen, die auch in einer für den Grundkörper des Werkzeugs oder Instruments aggressiven, korrodierenden Umgebung, insbesondere bei deren Verwendung als Schneidwerkzeuge für organische Materialien, als chirurgisches Instrument, als Instrument zur Maniküre, Pediküre, etc. sowie als Formwerkzeuge für Vulkanisier- oder Plastikspritzgußformen eine lange Lebensdauer bei verminderter Abrasion ergibt. Auch soll bei dem Werkzeug oder Instrument eine ansprechende Farbgestaltung der Oberfläche möglich sein, ohne die oben genannten vorteilhaften Eigenschaften zu verringern.

Die erfindungsgemäße Lösung der Aufgabe ist hinsichtlich des Werkzeugs oder Instruments durch die im Anspruch 1 und hinsichtlich des Verfahrens durch die im Anspruch 8 angegebenen Merkmale gekennzeichnet.

Die Ansprüche 2 bis 7 beschreiben bevorzugte Ausführungsformen des erfindungsgemäßen Werkzeugs oder Instruments und die Ansprüche 9 bis 11 bevorzugte Ausführungsarten des erfindungsgemäßen Verfahrens.

Im folgenden wird ein Beispiel des erfindungsgemäßen Werkzeugs oder Instruments und des erfindungsgemäßen Verfahrens zur Beschichtung des Werkzeugs oder Instruments anhand von zeichnerischen Darstellungen naher erläutert. Es zeigen:

Fig. 1    einen Schnitt durch eine schematische Darstellung einer die erfindungsgemäßen Beschichtung eines Werkzeugs oder Instruments,

Fig. 2    einen Schnitt durch eine schematisch dargestellte Aufdampfanlage,

Fig. 3    einen Schnitt durch die Aufdampfanlage in **Figur 2** entlang der Linie III - III, wobei aufgrund einer vorhandenen Rotationssymmetrie nur eine Hälfte der Aufdampfanlage dargestellt ist, und

Fig. 4    einen Schnitt durch eine Variante der in **Figur 1** dargestellten Beschichtung.

Unter organischem Material werden Materialien verstanden, welche durch die organischen Chemie beschrieben werden. Insbesondere werden hierunter tierische und pflanzliche Produkte sowie Kunststoffe verstanden, zu deren Bearbeitung u. a. Vulkanisierformen, Kunststoffspritzgußformen, Messer z. B. zur Bearbeitung von Fleisch gehören; auch Implantate, wie sie im menschlichen Körper verwendet werden, werden hierunter verstanden.

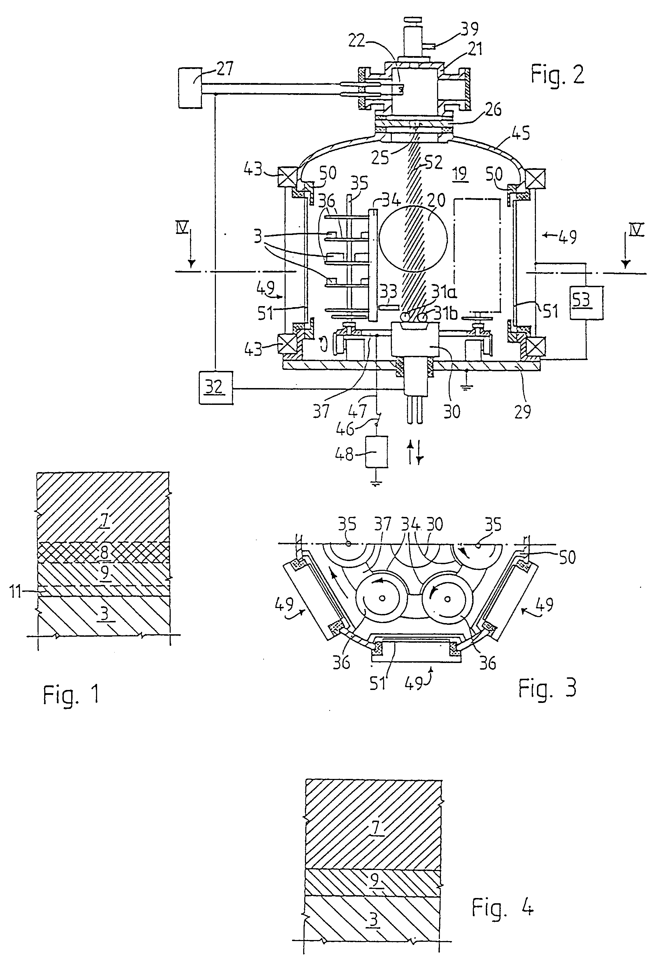

In einem Schnitt senkrecht zur Oberfläche des erfindungsgemäße Werkzeugs oder Instruments **1** ist in **Figur 1** dessen Grundkörper **3** mit einer Hartschicht **7** als Außenseite und eine dazwischenliegende Trennschicht **9** dargestellt. Die nach dem unten beschriebenen Verfahren aufgebrachte Trennschicht **9** ist röntgenamorph, d. h. sie zeigt bei Bestrahlung mit einem Röntgenstrahl auf der der Röntgenquelle abgewandten Seite der Trennschicht **9** nur eine annähernd gaußsche Intensitätsverteilung mit einem Strahlungsmaxima in der Strahlmitte; es ist keine Fernfeldordnung (kein Debye-Scherrer-Diagramm) zu erkennen.

Die Hartschicht **7** besteht im wesentlichen aus Titannitrid mit einer Schichtdicke von 3 $\mu$m.

Die Trennschicht **9** besteht aus einem keramischen, elektrisch nicht leitenden Material, dessen Schichtdicke je nach verwendetem Material und Einsatzgebiet zwischen 0,5 und 5 $\mu$m liegt. Unter einem keramischen Material wird eine chemische Verbindung verstanden, die in ihrer soliden Form als Keramik bekannt ist, wobei der Aufbau der Trennschicht **9** amorph ist. Als keramisches Material wird bevorzugt Aluminiumoxid $Al_2O_3$, wie unten beschrieben, verwendet.

Das Material der Trennschicht **9** geht in einer Übergangsschicht **8** allmählich in das Material der Hartschicht **7** über. Ebenfalls geht die Trennschicht **9** zum Grundkörper **3** hin allmählich in einer weiteren Übergangsschicht **11** in Aluminium, dem Bindungspartner des Aluminiumoxids des Materials der Trennschicht **9**, über.

**Figur 2** zeigt eine schematische Darstellung einer beispielsweisen Aufdampfanlage zur Durchführung der erfindungsgemäßen Beschichtung. Die Aufdampfanlage hat eine Vakuumkammer **19** mit einem Evakuierungsanschluß **20** und eine Glühkathodenkammer **21** mit einer Glühkathode **22**, die über eine Öffnung **25** mit der Vakuumkammer **19** verbunden ist. Der die Öffnung **25** beinhaltende Boden **26** der Glühkathodenkammer **21** ist gegenüber den Wänden der Vakuumkammer **19** elektrisch isoliert. Die Glühkathode **22** wird von einem Stromversorgungsgerät **27** gespeist. Unterhalb der Öffnung **25** befindet sich über dem Boden **29** der Vakuumkammer **19** ein Tiegel aus Titan, in dem Aluminium **31a** als Oxidbildner und Titan **31b** als Nitridbildner liegen. Der Titantiegel, sog. Titanliner, steht in einem kühlbaren Kupfertiegel. Diese Anordnung wird nachfolgend als Tiegel **30** bezeichnet. Der Titanliner sorgt einerseits für eine Wärmeisolation gegenüber den Kupfertiegel und erleichtert die Legierbarkeit von Aluminium mit Titan, um die Viskosität und die Reaktivität beim späteren Verfahrensablauf gegenüber Sauerstoff günstig zu beeinflussen. Das Aluminium **31a** und Titan **31b** sind mit einer verschiebbaren Blende **33** abdeckbar. In der Vakuumkammer **19** sind sechs um die Längsachse drehbare, elektrisch leitende Träger **35**, von denen vier in **Figur 3** angedeutet sind, vorhanden, an denen die zu beschichtenden Grundkörper **3** aus Stahl an je einer Halterung **36** gehalten werden. Die Träger **35** sind um ihre Achse drehbar auf einem Drehteller **37** angeordnet und durch diesen untereinander elektrisch verbunden. Der Drehteller **37** ist gegenüber dem Boden **29** und den Wänden der Vakuumkammer **19** elektrisch isoliert. Die Halterungen **36** sind mit den Trägern **35** elektrisch leitend verbunden. Die an den Halterungen **36** gehaltenen Grundkörper **3** sind mit einer in **Figur 2** und **3** schematisch dargestellten Blende **34** gegenüber dem Aluminium und Titan **31a** und **31b** im Tiegel **30** abdeckbar.

In die Glühkathodenkammer **21** mündet eine Gaszuleitung **39**, die über die Öffnung **25** mit der Vakuumkammer **19** verbunden ist. Je eine schematisch dargestellte Magnetspule **43** befindet sich gerade oberhalb des Bodens **29** und am Anschluß eines Deckelteils **45** der Vakuumkammer **19** zur Erzeugung eines annäherend parallelen vertikalen Magnetfelds.

Der Drehteller **37** ist über eine elektrische Leitung **47** und einen geschlossenen Schalter **46** mit einem einstellbaren Spannungsgenerator **48**, dessen anderer Pol geerdet ist, verbunden.

In den vertikalen Wänden der Vakuumkammer **19** sind sechs Vorrichtungen **49** zur Kathodenzerstäubung angeordnet, von denen drei in **Figur 3** dargestellt sind. Die Vorrichtung **49** ist mit einem nicht dargestellten Wärmetauscher zur Kühlung versehen. Innerhalb eines Rings **50** liegt ein von ihm isoliertes Target **51**, welches mit dem negativen Pol einer Spannungsquelle **53** verbunden ist. Der positive Pol der Spannungsquelle **53** ist mit den Wänden der Vakuumkammer **19** und dem Ring **50** verbunden. Die Glühkathode **22** und der Tiegel **30** sind über elektrische Leitungen mit einem Stromversorgungsgerät **32** verbunden.

Zur Herstellung der obigen Beschichtung werden die Grundkörper **3** an den Halterungen **36** der Träger **35** befestigt und Aluminium und Titan **31a** und **31b** in den Tiegel **30** gelegt. Anschließend wird die Vakuumkammer **19** geschlossen, auf einen Druck von 2 mPa evakuiert und die zu beschichtenden Oberflächen der Gegenstände **3** entsprechend einem in der DE-OS 34 06 953 bzw. der CH-PS 658 545 beschriebenen Verfahren mit einem Niedervoltbogen **52** auf 450 °C erhitzt und entsprechend einem in der CH-PS 631 743 beschrieben Verfahren gereinigt. Die Blende **33** bedeckt währenddessen das Aluminium und Titan **31a** und **31b** im Tiegel **30**.

In der Vakuumkammer **19** wird Argon eingeleitet und dessen Druck auf 0,15 Pa eingestellt. Die Blende **33** wird entfernt und der Niedervoltbogen **52** zum Tiegel **30** gezündet. Bei einem Strom des Niedervoltbogens **52** von 80 A erfolgt ein Legieren von Aluminium **31a** und Titan **31b** ohne eine wesentliche Verdampfung des Aluminiums. Nach etwa vier Minuten ist dieser Vorgang abgeschlossen, die Blende **34** vor den Trägern **35** wird weggeklappt und der Strom des Niedervoltbogens **52** wird auf 120 A erhöht, um Aluminium zu verdampfen. Aufgrund des hohen Unterschiedes von fünf Größenordnungen der Dampfdrücke von Aluminium und Titan wird kein Titan verdampft. Innerhalb der nächsten vier Minuten wird Aluminium ohne Zusatz eines Reaktivgases verdampft und direkt auf der Oberfläche des Grundkörpers **3** als unterster Bereich der Übergangsschicht **11** niedergeschlagen.

Innerhalb der nun folgenden zwei Minuten wird Sauerstoff durch die Gaszuleitung **39** mit steigender Zuflußmenge eingelassen und ein Gesamtdruck in der Vakuumkammer **19** von 0,4 Pa eingestellt. Der Sauerstoff wird durch den Niedervoltbogen **52** teilweise ionisiert. Der teilweise ionisierte Sauerstoff und das teilweise ionisierte Aluminium vereinigen sich auf der Oberfläche des Grundkörpers zu $Al_2O_3$ und bleiben auf ihr haften. Je nach der vorhanden Menge Sauerstoff ändert sich das Verhältnis der niedergeschlagenen Menge Aluminiumoxid zum Aluminium. Der Sauerstoffzufluß beträgt bei obigem Enddruck 200 Standardkubikzentimeter pro Minute. Damit die Verdampfungsrate des Aluminiums durch dessen Verbrauch nicht absinkt, wird durch eine nicht dargestellte Stromregelung der Strom des Niedervoltbogens **52** hochgeregelt. Innerhalb der folgenden fünfzehn Minuten ist der Strom des Niedervoltbogens **52** bis auf 200 A hochgeregelt und ein wesentlicher Teil des Aluminiums verdampft.

Ist eine Schichtdicke der Trennschicht von 1 $\mu$m aufgebracht wird die Sauerstoffzufuhr derart stetig reduziert und eine Stickstoffzufuhr derart stetig erhöht, daß obiger Druck von 0,4 Pa in der Vakuumkammer **19** erhalten bleibt. Dieser Verfahrensschritt dauert fünf Minuten und die Übergangsschicht **8** aus $Al_xTi_yO_uN_v$ zwischen der Trennschicht **9** und der noch herzustellenden Hartschicht **7** ist fertig, wobei die Werte für x und u mit wachsender Übergangsschicht **8** ab- und die Werte für y und v zunehmen. Die Werte für v werden etwa gleich den Werten von y und die Werte von u etwa $(3/2) \cdot x$ gewählt.

Während nun folgender dreißig Minuten wird mit dem Niedervoltbogen **52** Titan aus dem Tiegel **30** in der Stickstoffatmosphäre verdampft und auf der Übergangsschicht **8** die Titannitrid-Hartschicht **7** niedergeschlagen. Danach wird der Niedervoltbogen **52** und die Stickstoffzufuhr abgeschaltet.

Anstelle einer Aluminiumoxidschicht $Al_2O_3$ als Trennschicht **9** kann auch eine Siliziumnitridschicht $Si_3N_4$ aufgebracht werden. Hierbei wird nur Silizium in den Tiegel **30** gegeben. Der Niedervoltbogen **52** wird gezündet und brennt von der Glühkathode **22** zum Silizium im Tiegel **30** mit einer Bogenspannung von 90 V und einem Strom von 60 A, wobei bei Erreichen des Schmelzpunktes die Spannung auf 70 V abfällt und der Strom auf 200 A aufgrund der sich mit der Temperatur erhöhenden Leitfähigkeit des Siliziums ansteigt.

In einem folgenden ersten Verfahrensschritt wird die Stromstärke des Niedervoltbogens **52** auf 200 A bei einer Bogenspannung von 70 V gehalten. Hierdurch wird das Silizium aus dem Tiegel **30** in den gasförmigen Zustand überführt und teilweise ionisiert.

In einem zweiten Verfahrensschritt wird in die beim Reinigungsvorgang verwendete Argonatmosphäre Stickstoff durch die Gaszuleitung **39** eingelassen, der durch den Niedervoltbogen **52** teilweise ionisiert wird. Gleichzeitig wird die Blende **34** vor den Grundkörpern **3** weggeklappt. Der teilweise ionisierte Stickstoff und das teilweise ionisierte Silizium vereinigen sich auf der Oberfläche der Grundkörper **3** zu $Si_3N_4$ und bleiben auf ihr haften. Auch während dieses Verfahrensschritts rotieren die Grundkörper **3**.

Während dieses zweiten Verfahrensschrittes beträgt der Partialdruck des Argons 0,4 Pa und der des Stickstoffs 0,3 Pa. An den Trägern **35** liegt eine pulsierende Gleichspannung mit einer Periodendauer von 10 μs. Zu Beginn dieses Verfahrensschrittes liefert der Spannungsgenerator **48** negative Pulse mit einer Pulsbreite von 8 μs und einer Amplitude von - 200 V. In den folgenden 2 μs sind die Träger **35** über den Spannungsgenerator **48** geerdet. Während dieses Verfahrensschrittes wird die Amplitude derart zu kleineren negativen Werten verändert, daß sie gegen Ende bei - 10 V liegt.

Es kann auch das Material der keramischen Trennschicht **9** mit einem Elektronenstrahl, durch eine Kathodenzerstäubung (Sputtern), eine plasmaunterstütze Verdampfung oder eine kathodische Bogenverdampfung in den gasförmigen Zustand überführt werden. Die Bogenentladung kann neben den Niedervoltbogen mit einem heißen Filament, mit Funkenverdampfung oder Hohlkathode arbeiten.

Es kann auch eine Trennschicht **9** aus einem Oxid und/oder Nitrid eines Elements der Gruppe IVb (Titan, Zirkon, Hafnium), Vb (Vanadium, Niob, Tantal), VIb (Chrom, Molybdän, Wolfram) des Periodensystems oder aus Mischungen dieser Stoffe oder Mischungen dieser Stoffe mit einem Oxid, Nitrid oder Oxynitrids des Aluminiums erfolgen.

Anstelle den nicht mit der Leitung **47** verbundenen Pol des Spannungsgenerators **48** zu erden, kann er auch an den Tiegel **30** oder an Anodenpotential des Niedervoltbogens **52** angeschlossen werden. Anstelle die Pulshöhe von - 200 V auf - 10 V zu verändern, kann auch die Pulsbreite verkleinert werden; es kann auch die Pulshöhe und die Pulsbreite verändert werden.

Anstelle die Hartschicht **(7)** durch Ionenplattieren aufzubringen, kann sie auch aufgesputtert werden. Hierzu werden Targets **51** aus Titan ausgebildet, im Tiegel **30** befindet sich in diesem Fall nur Silizium. Ist eine Schichtdicke der Trennschicht **9** von 1 μm aufgebracht, wird das im Tiegel **30** befindliche Silizium abgedeckt, der Niedervoltbogen **52** und der Spannungsgenerator **48** ausgeschaltet, der Schalter **46** geöffnet und der Stickstoffzufluß auf einen Zufluß von 250 Standardkubikzentimetern pro Minute eingestellt. Argon wird in die Vakuumkammer **19** bis zum Erreichen einer Druckes von 0,8 Pa eingelassen, an das Target **51** (Größe 12,7 x 45,72 cm (5 x 18 Zoll)) mit der Spannungsquelle **53** eine Spannung von 650 V gelegt, wodurch eine Sputterleistung von 10kW pro Target **51** sich ergibt. Die Grundkörper **3** werden an eine Gleichspannung von - 80 V gelegt. Der Stickstoffzufluß wird derart kontrolliert, daß keine Targetvergiftung erfolgt. Ist innerhalb einer halben Stunde eine Titannitridschichtdicke von 2 μm auf der Trennschicht **9** niedergeschlagen, wird die Spannungsquelle **53** ausgeschaltet. Die Vakuumkammer **19** wird geflutet und anschließend zur Entnahme der beschichteten Werkstücke oder Instrumente **1** geöffnet.

Die Schichtdicke der Hartschicht **7** wird für allgemein bekannte Anwendung mit einer Dicke, die zwei- bis zehnmal der Schichtdicke der Trennschicht **9** entspricht, vorgenommen.

Anstelle von Titannitrid lassen sich auch andere Materialien als Hartschicht **7**, die im wesentlichen Verbindungen des Stickstoffs, Kohlenstoffs oder Bors mit einem Metall der Gruppe IVb (Titan, Zirkon, Hafnium), Vb (Vanadium, Niob, Tantal) oder VIb (Chrom, Molybdän, Wolfram) des Periodensystems oder Siliziumkarbid oder deren Mischungen oder Titannitrid und/oder Titankarbonitrid enthalten, verwenden.

Je nach verwendeten Materialien werden neben Sputtern auch andere PVD-Verfahren, wie z. B. reaktives PVD zum Aufbringen der Hartschicht verwendet.

Aufgrund der niedrigen Temperaturen, die der Grundkörper **3** während des gesamten Beschichtungsvorgangs ausgesetzt ist, lassen sich Wärmebehandlungen vor dem Beschichtungsvorgang am Grundkörper **3** durchführen, ohne befürchten zu müssen, daß sie sich verziehen.

Bei den erfindungsgemäß beschichteten Grundkörpern **3** erhöht die keramische Trennschicht namentlich die Korrosionbeständigkeit drastisch. Dieses Ergebnis ist namentlich insofern überraschend, als bis jetzt bei mittels PVD aufgebrachten Schichten gewöhnlich "pin holes", die einer Korrosionsbeständigkeit entgegenwirken, beobachtet wurden.

Das erfindungsgemäße Werkzeug oder Instrument eignet sich aufgrund seiner hervorragenden Korrosionsbeständigkeit und aufgrund seines hohen Abrasionswiderstand insbesondere zur Verwendung als Schneidwerkzeuge für organische Produkte und Kunststoffe, als chirugisches Instrument, als Implantat im menschlichen Körper, als Instrument zur Maniküre, Pediküre, etc. sowie als Formwerkzeuge für Vulkanisier- oder Kunststoffspritzgußformen.

**Patentansprüche**

1. Beschichtetes Werkzeug oder Instrument **(1)** mit einem Grundkörper **(3)** und einer Beschichtung mit mindestens einer verschleißresistenten, eine Härte größer 1500 Vickers aufweisenden Hartschicht **(7)** an der Außenseite der Beschichtung zum Be- oder Verarbeiten von organischem Material, **dadurch gekennzeichnet**, daß
die im wesentlichen eine Verbindung des Stickstoffs, Kohlenstoffs oder Bors mit einem Metall der Gruppe IVb (Ti, Zr, Hf), Vb (V, Nb, Ta) oder VIb (Cr, Mo, W) des Periodensystems oder Siliziumkarbid oder deren Mischungen oder Titannitrid und/oder Titancarbonitrid enthaltende Hartschicht **(7)**
vom Grundkörper **(3)** durch eine mittels reaktivem PVD-Verfahren abgeschiedene,
röntgenamorphe,
keramische,
elektrisch nicht leitende,
im wesentlichen aus einem Oxid und/oder Nitrid eines Elements der Gruppe IVb (Ti, Zr, Hf), Vb (V, Nb, Ta), VIb (Cr, Mo, W) des Periodensystems oder aus Mischungen dieser Stoffe oder Mischungen dieser Stoffe mit einem Oxid, Nitrid oder Oxinitrids des Aluminiums bestehende Trennschicht **(9)** getrennt ist.

2. Werkzeug oder Instrument **(1)** nach Anspruch 1, **dadurch gekennzeichnet**, daß
die Trennschicht **(9)** eine Schichtdicke von annähernd 0,5 bis 5 $\mu$m hat.

3. Werkzeug oder Instrument **(1)** nach Anspruch 1 oder 2, **dadurch gekennzeichnet**, daß
die Hartschicht **(7)** die zwei- bis zehnfache Dicke der Trennschicht **(9)** hat.

4. Werkzeug oder Instrument **(1)** nach einem der Ansprüche 1 bis 3, **gekennzeichnet durch**
ein unmittelbar auf dem Grundkörper **(3)** befindliches Material des Bindungspartners des Oxids, Nitrids oder Oxinitrids der Trennschicht **(9)**, wobei
die Materialzusammensetzung von der Oberfläche des Grundkörpers **(3)** allmählich in diejenige der Trennschicht **(9)** übergeht.

5. Werkzeug oder Instrument **(1)** nach einem der Ansprüche 1 bis 4 , **gekennzeichnet durch**
eine weitere Übergangsschicht **(8)** zwischen der Hart-**(7)** und der Trennschicht **(9)**, in der das Trennschichtmaterial allmählich in das Hartschichtmaterial, bevorzugt im wesentlichen Titannitrid, übergeht.

6. Werkzeug oder Instrument **(1)** nach einem der Ansprüche 1 bis 3, **dadurch gekennzeichnet**, daß
die Trennschicht **(9)** unmittelbar auf der Oberfläche des Grundkörpers **(3)** und die Hartschicht **(7)** unmittelbar auf der Trennschicht **(9)** liegt.

7. Werkzeug oder Instrument (1) nach einem der Ansprüche 1 bis 6, **dadurch gekennzeichnet**, daß
die Oberfläche des Grundkörpers **(3)** wärmebehandelt ist.

8. Verfahren zur Herstellung eines beschichteten Grundkörpers eines Werkzeugs oder Instruments gemäß einem der Patentansprüche 1 bis 7 mit einer Beschichtung mit einem keramischen, elektrisch nicht leitenden Material auf einer Oberfläche eines gegenüber einer Vakuumkammer **(19)** isoliert gehaltenen Grundkörpers **(3)**, wobei
in einem ersten Verfahrensschritt ein Oxid- und/oder Nitridbildner **(31)** aus der Gruppe IVb, Vb, VIb des Periodensystems oder Mischungen dieser Stoffe in den gasförmigen Zustand übergeführt und wenigstens teilweise ionisiert wird,
in einem zweiten Verfahrensschritt ein Reaktionsgas im wesentlichen Stickstoff und/oder Sauerstoff in die Vakuumkammer **(19)** eingeleitet und wenigstens teilweise ionisiert wird, worauf
sich auf der Oberfläche des Grundkörpers **(3)** eine keramische, elektrisch nicht leitende, röntgenamorphe Trennschicht **(9)** aus einer Verbindung des Oxid- und/oder Nitridbildners und des Stickstoffs und/oder Sauerstoffs niederschlägt, und
in einem dritten Verfahrensschritt eine verschleißresistente Hartschicht **(7)** als Verbindung des Stickstoffs, Kohlenstoffs oder Bors mit einem Metall der Gruppe IVb, Vb oder VIb des Periodensystems oder Siliziumkarbid oder deren Mischungen oder Titannitrid und/oder Titancarbonitrid niedergeschlagen wird, wobei
zumindest während des zweiten Verfahrensschritts eine pulsierende Gleichspannung an den Grundkörper und/oder dessen Halter gelegt wird.

9. Verfahren nach Anspruch 8, **dadurch gekennzeichnet**, daß
die Pulshöhe der pulsierenden Gleichspannung beginnend mit einem hohen negativen Spannungswert auf einen geringen negativen Wert verändert und
zwischen den Pulsen der Grundkörper **(3)** und/oder dessen Halter **(36)** geerdet oder auf das Potential des noch nicht in den gasförmigen Zustand überführten Oxid- und/oder Nitridbildners **(31)** oder auf Anodenpotential einer Bogenentladung **(52)** zur Überführung des Oxid- und/oder Nitridbildners **(31)** in den gasförmigen Zustand gelegt wird.

10. Verfahren nach Anspruch 8 oder 9, **dadurch gekennzeichnet**, daß
zur Überführung des Oxid- und/oder Nitridbildners in den gasförmigen Zustand und zu dessen wenigstens teilweiser Ionisierung sowie zur wenigstens teilweisen Ionisierung des Stickstoffs und/oder Sauerstoffs eine Bogenentladung **(52)** verwendet wird.

11. Verfahren nach Anspruch 10, **dadurch gekennzeichnet**, daß
im ersten Verfahrensschritt Aluminium mit Titan zusammen in einem Titantiegel mit einer Bogenentladung geschmolzen werden,
nach dem Schmelzen der Bogenentladungsstrom erhöht wird, bis Aluminium verdampft,
anschließend im zweiten Verfahrensschritt im wesentlichen Sauerstoff als Reaktionsgas zugeführt wird,
der Bogenentladungsstrom kontinuierlich erhöht wird, bis ein wesentlicher Teil des Aluminiums verdampft ist und das Titan zu verdampfen beginnt, wobei
bei Verdampfungsbeginn des Titans Stickstoff als weiteres Reaktionsgas zugeführt und die Zufuhr in dem Maße erhöht wird, wie die Sauerstoffzufuhr reduziert wird, um
im dritten Verfahrensschritt auf der Trennschicht **(9)** die Hartschicht **(7)** aus Titannitrid mit einem allmählichen Übergang zwischen den Materialien der Trennschicht **(9)** und der Hartschicht **(7)** zu erzeugen.

**Claims**

1. A coated tool or instrument (1) having a base body (3) and a coating with at least one wear-resistant hard layer (7) of a hardness of greater than 1500 Vickers at the outside of the coating for working or processing organic material, characterised in that
the hard layer (7) which essentially contains a compound of nitrogen, carbon or boron with a metal of group IVb (Ti, Zr, Hf), Vb (V, Nb, Ta) or VIb (Cr, Mo, W) of the periodic system or silicon carbide or mixtures thereof or titanic nitride and/or titanic carbonitride
is separated from the base body (3) by a separation layer (9) which is deposited by bans of a reactive PVD-process and

is X-ray amorphous,
ceramic,
electrically non-conducting and
essentially comprises an oxide and/or nitride of an element of group IVb (Ti, Zr, Hf), Vb (V, Nb, Ta), VIb (Cr, Mo, W) of the periodic system or mixtures of said substances or mixtures of said substances with an oxide, nitride or oxynitride of aluminium.

2. A tool or instrument (1) according to claim 1 characterised in that
the separation layer (9) is of a layer thickness of approximately 0.5 to 5 $\mu$m.

3. A tool or instrument (1) according to claim 1 or claim 2 characterised in that
the hard layer (7) is twice to ten times the thickness of the separation layer (9).

4. A tool or instrument (1) according to one of claims 1 to 3 characterized by
a material, disposed directly on the base body (3), of the bond partner of the oxide, nitride or oxynitride of the separation layer (9), wherein
the material composition gradually goes from the surface of the base body (3) into that of the separation layer (9).

5. A tool or instrument (1) according to one of claims 1 to 4 characterised by
a further transition layer (8) between the hard layer (7) and the separation layer (9), in which the separation layer material gradually goes into the hard layer material, preferably essentially titanium nitride.

6. A tool or instrument (1) according to one of claims 1 to 3 characterised in that
the separation layer (9) lies directly on the surface of the base body (3) and the hard layer (7) lies directly on the separation layer (9).

7. A tool or instrument (1) according to one of claims 1 to 6 characterised in that
the surface of the base body (3) is heat-treated.

8. A process for the production of a coated base body of a tool or instrument according to one of claims 1 to 3 with a coating with a ceramic, electrically non-conducting material on a surface of a base body (3) which is kept insulated relative to a vacuum chamber (19), wherein
in a first process step an oxide and/or nitride-forming agent (31) from the group IVb, Vb and VIb of the periodic system or mixtures of said substances is put into the gaseous state and at least partially ionised,

in a second process step a reaction gas, essentially nitrogen and/or oxygen, is introduced into the vacuum chamber (19) and at least partially ionised, whereupon

a ceramic, electrically non-conducting, X-ray amorphous separation layer (9) comprising a compound of the oxide and/or nitride-forming agent and the nitrogen and/or oxygen is deposited on the surface of the base body (3), and

in a third process step a wear-resistant hard layer (7) as a compound of nitrogen, carbon or boron with a metal of group IVb, Vb or VIb of the periodic system or silicon carbide or mixtures thereof or titanium nitride and/or titanium carbonitride is deposited, wherein

at least during the second process step a pulsating dc voltage is applied to the base body and/or the holder thereof.

9. A process according to claim 8 characterised in that

the pulse height of the pulsating dc voltage, beginning with a high negative voltage value, is changed to a low negative value, and

between the pulses the base body (3) and/or the holder (36) thereof is earthed or is set to the potential of the oxide and/or nitride-forming agent (31) which has not yet been put into the gaseous state or to anode potential of an arc discharge (52) for putting the oxide and/or nitride-forming agent (31) into the gaseous state.

10. A process according to claim 8 or claim 9 characterised in that

for putting the oxide and/or nitride-forming agent into the gaseous state and for the at least partial ionisation thereof and also for the at least partial ionisation of the nitrogen and/or oxygen, an arc discharge (52) is used.

11. A process according to claim 10 characterised in that

in a first process step aluminium is melted together with titanium in a titanium crucible by an arc discharge,

after the melting operation the arc discharge current is increased until aluminium vaporises,

then in the second process step essentially oxygen is supplied as the reaction gas,

the arc discharge current is continuously increased until a substantial part of the aluminium is vaporised and the titanium begins to vaporise, wherein

with the beginning of vaporisation of the titanium nitrogen is supplied as a further reaction gas and the supply is increased to the extent to which the oxygen supply is reduced in order

in the third process step to produce on the separation layer (9) the hard layer (7) comprising titanium nitride with a gradual transition between the materials of the separation layer (9) and the hard layer (7).

**Revendications**

1. Outil ou instrument revêtu (1) formé d'un corps de base (3) et d'un revêtement contenant au moins une couche dure (7) résistante à l'usure et présentant une dureté supérieure à 1500 Vickers, sur la face extérieure du revêtement, pour le traitement ou la transformation de matériaux organiques, caractérisé en ce que la couche dure (7) contenant essentiellement une combinaison d'azote, de carbone ou de bore avec un métal du groupe IVb (Ti, Zr, Hf), Vb (V, Nb, Ta) ou VIb (Cr, Mo, W) de la classification périodique, ou du carbure de silicium ou des mélanges de ceux-ci ou du nitrure de titane et/ou du carbonitrure de titane, est séparée du corps de base (3) par une couche de séparation (9) extraite à l'aide d'un procédé PVD réactif, amorphe aux rayons X, céramique et non électroconductrice formée essentiellement d'un oxyde et/ou d'un nitrure d'un élément du groupe IVb (Ti, Zr, Hf), Vb (V, Nb, Ta), VIb (Cr, Mo, W) de la classification périodique ou de mélanges de ces substances ou de mélanges de ces substances avec un oxyde, un nitrure ou un oxynitrure d'aluminium.

2. Outil ou instrument (1) selon la revendication 1, caractérisé en ce que la couche de séparation (9) a une épaisseur d'environ 0,5 à 5 $\mu$m.

3. Outil ou instrument (1) selon la revendication 1 ou 2, caractérisé en ce que la couche dure (7) a une épaisseur égale à deux à dix fois celle de la couche de séparation (9).

4. Outil ou instrument (1) selon l'une des revendications 1 à 3, caractérisé par un matériau, situé directement sur le corps de base (3), du composant de l'oxyde, du nitrure ou de l'oxynitrure de la couche de séparation (9), étant précisé que la composition du matériau passe progressivement de la surface du corps de base (3) à celle de la couche de séparation (9).

**5.** Outil ou instrument (1) selon l'une des revendications 1 à 4, caractérisé par une autre couche de transition (8) prévue entre la couche dure (7) et la couche de séparation (9) et dans laquelle le matériau de la couche de séparation se transforme progressivement en matériau de la couche dure, de préférence essentiellement du nitrure de titane.

**6.** Outil ou instrument (1) selon l'une des revendications 1 à 3, caractérisé en ce que la couche de séparation (9) se trouve directement sur la surface du corps de base (3) et la couche dure (7) se trouve directement sur la couche de séparation (9).

**7.** Outil ou instrument (1) selon l'une des revendications 1 à 6, caractérisé en ce que la surface du corps de base (3) subit un traitement thermique.

**8.** Procédé pour la fabrication d'un corps de base revêtu d'un outil ou d'un instrument selon l'une des revendications 1 à 7, formé d'un revêtement contenant un matériau céramique non électroconducteur sur une surface d'un corps de base (3) maintenu isolé par rapport à une chambre à vide (19), selon lequel

lors d'une première phase du procédé, un générateur d'oxyde et/ou de nitrure (31) du groupe IVb, Vb, VIb de la classification périodique ou des mélanges de ces substances sont transformés en état gazeux et au moins partiellement ionisés,

lors d'une deuxième phase du procédé, un gaz de réaction, essentiellement de l'azote et/ou de l'oxygène, est introduit dans la chambre à vide (19) et au moins partiellement ionisé,

après quoi une couche de séparation (9) céramique, non électroconductrice et amorphe aux rayons X formée d'une combinaison du générateur d'oxyde et/ou de nitrure et de l'azote et/ou de l'oxygène se dépose sur la surface du corps de base (3), et

lors d'une troisième phase du procédé, une couche dure (7) résistante à l'usure se dépose sous la forme d'une combinaison d'azote, de carbone ou de bore avec un métal du groupe IVb, Vb ou VIb de la classification périodique ou du carbure de silicium et des mélanges de ceux-ci ou du nitrure de titane et/ou du carbonitrure de titane,

étant précisé qu'au moins pendant la deuxième phase du procédé, une tension continue pulsée est appliquée au corps de base et/ou à l'attache de celui-ci.

**9.** Procédé selon la revendication 8, caractérisé en ce que la hauteur d'impulsion de la tension continue pulsée varie, à partir d'une valeur de tension négative élevée, pour atteindre une valeur négative faible, et

entre les impulsions, le corps de base (3) et/ou son attache (36) sont mis à la terre ou au potentiel du générateur d'oxyde et/ou de nitrure (31) pas encore transformé en état gazeux, ou au potentiel anodique d'une décharge en arc (52) pour la transformation du générateur d'oxyde et/ou de nitrure (31) en état gazeux.

**10.** Procédé selon la revendication 8 ou 9, caractérisé en ce que pour la transformation du générateur d'oxyde et/ou de nitrure en état gazeux et pour son ionisation au moins partielle, ainsi que pour l'ionisation au moins partielle de l'azote et/ou de l'oxygène, on utilise une décharge en arc (52).

**11.** Procédé selon la revendication 10, caractérisé en ce que

lors de la première phase du procédé, de l'aluminium et du titane sont fondus ensemble dans un creuset en titane à l'aide d'une décharge en arc,

après la fusion, le courant de la décharge en arc est augmenté jusqu'à ce que l'aluminium s'évapore,

puis, lors de la deuxième phase du procédé, on introduit essentiellement de l'oxygène comme gaz de réaction,

le courant de la décharge en arc est continuellement augmenté jusqu'à ce qu'une proportion importante d'aluminium soit évaporée et que le titane commence à s'évaporer,

étant précisé qu'au début de l'évaporation du titane, de l'azote est amené comme second gaz de réaction et que cette amenée est augmentée proportionnellement à la réduction de l'amenée d'oxygène,

afin de générer sur la couche de séparation (9), lors de la troisième phase du procédé, la couche dure (7) de nitrure de titane avec une transformation progressive entre les matériaux de le couche de séparation (9) et la couche dure (7).

Fig. 2

Fig. 1

Fig. 3

Fig. 4